(19) Europäisches Patentamt
European Patent Office
Office européen des brevets

(11) Veröffentlichungsnummer: **0 181 571**
**A1**

(12) **EUROPÄISCHE PATENTANMELDUNG**

(21) Anmeldenummer: 85113841.2

(22) Anmeldetag: 30.10.85

(51) Int. Cl.⁴: C 07 D 499/00
A 61 K 31/43
//C07D205/08, C07F9/65

(30) Priorität: 02.11.84 CH 5266/84

(43) Veröffentlichungstag der Anmeldung:
21.05.86 Patentblatt 86/21

(84) Benannte Vertragsstaaten:
AT BE CH DE FR GB IT LI LU NL SE

(71) Anmelder: CIBA-GEIGY AG
Klybeckstrasse 141
CH-4002 Basel(CH)

(72) Erfinder: Schneider, Peter, Dr.
Bäumliackerstrasse 8
CH-4103 Bottmingen(CH)

(74) Vertreter: Zumstein, Fritz, Dr. et al,
Bräuhausstrasse 4
D-8000 München 2(DE)

(54) **Kristalline Aminomethyl-Verbindung.**

(57) Die Erfindung betrifft (5R,6S)-2-Aminomethyl-6-[(1R)-1-hydroxyäthyl]-2-penem-3-carbonsäure in kristalliner Form und ein Verfahren zur Herstellung derselben. Die Substanz ist zur Behandlung von Infektionserkrankungen geeignet.

EP 0 181 571 A1

Croydon Printing Company Ltd

0181571

CIBA-GEIGY AG
Basel (Schweiz)

4-15133/+

## Kristalline Aminomethyl-Verbindung

Die Erfindung betrifft die (5R,6S)-2-Aminomethyl-6-[(1R)-1-hydroxyäthyl]-2-penem-3-carbonsäure in kristalliner Form, Verfahren zur Herstellung derselben und diese Verbindung enthaltende pharmazeutische Präparate.

In der Deutschen Offenlegungsschrift Nr. 2950898 ist die racemische (5R,6S,1'R und 5S,6R,1'S)-2-Aminomethyl-6-(1'-hydroxyäthyl)-2-penem-3-carbonsäure, ein Verfahren zu ihrer Herstellung und ihre Verwendung als antibakterielles Mittel beschrieben. Die racemische Verbindung fällt bei der Aufarbeitung als amorphe Substanz an.

Amorphe Substanzen sind mit verschiedenen Nachteilen behaftet, die sie zur Verwendung, insbesondere zur Herstellung von pharmazeutischen Präparaten, als wenig geeignet oder sogar ungeeignet erscheinen lassen. Ein Nachteil ist in der, verglichen mit kristallisierten Substanzen, relativ grossen Oberfläche der amorphen Substanzen zu sehen, die, verbunden mit der regellosen thermodynamisch ungünstigen Anordnung der Moleküle im Festkörper, für eine beträchtlich grössere Anfälligkeit gegenüber äusseren Einflüssen, wie Luft, Licht und erhöhte Temperatur, verantwortlich ist. Weiterhin neigen amorphe Substanzen in weit grösserem Ausmass als kristallisierte Verbindungen dazu, Lösungsmittel einzuschliessen und sich der Abgabe dieser Verunreinigungen, beispielsweise bei der Trocknung, hartnäckig zu widersetzen. Derartige Verunreinigungen, insbesondere toxische Lösungsmittel wie Aceton oder Methanol, enthaltende Präparate sind für die medizinische Verwendung, insbesondere bei parenteraler Applikation, nicht geeignet. Ein weiterer Nachteil amorpher Produkte liegt darin, dass Luftfeuchtigkeit in erheblich grösserem Masse, als

dies bei kristallinen Produkten der Fall ist, aufgenommen wird. Der steigende Wassergehalt solcher Produkte erschwert einerseits die Herstellung von pharmazeutischen Präparaten mit einem konstanten Wirkstoffgehalt und hat darüberhinaus einen negativen Einfluss auf die Rieselfähigkeit des Produktes. Amorphe Produkte besitzen ein relativ grosses Schüttvolumen, was die Verwendung grösserer Gefässe bei der Lagerung und bei der Herstellung von Arzneimittelzubereitungen erforderlich machen kann. Das oft mangelhafte Lösungsverhalten von amorphen Produkten (diese "verpappen" oder verkleben leicht, wodurch sich die Auflösungsgeschwindigkeit verringert) soll in diesem Zusammenhang ebenfalls erwähnt werden.

Die genannten Nachteile von amorphen Produkten, insbesondere die geringe Lagerstabilität, lassen es wünschenswert erscheinen, eine von der amorphen Form verschiedene Aggregatform aufzufinden, welche die für einen Arzneiwirkstoff zu fordernden Eigenschaften, wie insbesondere gute Lagerstabilität und Gewichtskonstanz, aufweist.

Es wurde nun gefunden, dass kristalline, optisch aktive (5R,6S)-2-Aminomethyl-6-[(1R)-1-hydroxyäthyl]-2-penem-3-carbonsäure, neben Vorteilen, die auf den kristallinen Zustand zurückzuführen sind, überraschende pharmakologische Eigenschaften aufweist.

Die Erfindung betrifft demgemäss (5R,6S)-2-Aminomethyl-6-[(1R)-1-hydroxyäthyl]-2-penem-3-carbonsäure in kristalliner Form.

Unter der Bezeichnung "kristallin" ist zu verstehen, dass das Produkt im wesentlichen frei von amorphen Bestandteilen ist.

Insbesondere betrifft die Erfindung die kristalline (5R,6S)-2-Aminomethyl-6-[(1R)-1-hydroxyäthyl]-2-penem-3-carbonsäure, gekennzeichnet durch die nachfolgenden Gitterabstände (d-Werte) und relativen Linienintensitäten ihres Röntgenpulverdiagramms (Kamera nach Guinier, Strahlungsquelle Kupfer-$K_{\alpha 1}$):

| d-Werte (Angstroem) | relative Intensität |
|---|---|
| 10.9 | sehr stark |
| 10.0 | mittel |
| 9.7 | mittel |
| 7.9 | stark |
| 7.3 | stark |
| 7.0 | stark |
| 6.7 | schwach |
| 6.3 | sehr stark |
| 5.90 | sehr schwach |
| 5.85 | sehr schwach |
| 5.60 | sehr schwach |
| 5.53 | sehr schwach |
| 5.44 | sehr schwach |
| 5.33 | sehr schwach |
| 5.01 | sehr schwach |
| 4.93 | schwach |
| 4.64 | sehr schwach |
| 4.58 | sehr schwach |
| 4.53 | stark |
| 4.42 | sehr schwach |
| 4.34 | sehr stark |
| 4.28 | sehr schwach |
| 4.23 | sehr schwach |
| 4.10 | stark |
| 4.05 | sehr schwach |
| 3.99 | mittel |
| 3.91 | schwach |
| 3.82 | mittel |
| 3.78 | mittel |
| 3.73 | stark |
| 3.65 | mittel |
| 3.59 | stark |
| 3.54 | mittel |
| 3.42 | stark |
| 3.36 | mittel |
| 3.29 | stark |
| 3.20 | mittel |
| 3.13 | mittel |
| 3.11 | stark |
| 3.03 | mittel |
| 3.00 | mittel |

Die kristalline (5R,6S)-2-Aminomethyl-6-[(1R)-1-hydroxyäthyl]-2-penem-3-carbonsäure enthält pro Mol etwa 0,2 bis 1,5 Mol Wasser. Auf Grund der Feststellung, dass das Röntgenpulverdiagramm durch den wechselnden Wassergehalt nur unwesentlich beeinflusst wird (die Gitterabstände bleiben konstant und die geschätzten Linienintensitäten variieren nur sehr geringfügig), muss angenommen werden, dass das

Wasser nicht Teil des Kristallgitters, sondern im Kristallverbund mehr oder weniger locker gebunden ist. Ein definiertes Hydrat liegt daher nicht vor.

Das Produkt weist eine gute Kristallinität auf, ist auch bei längerer Einwirkung von Licht, Wärme (50°C) und Luft sehr stabil und zeigt bei annähernd normalen Umgebungsbedingungen keine Tendenz, grössere Mengen von Wasser aus der Luft aufzunehmen. Die Lagerstabilität ist somit als gut zu bezeichnen. Im Vergleich zu der vorbekannten amorphen, racemischen 2-Aminomethyl-6-[(1R)-1-hydroxyäthyl]-2-penem-3-carbonsäure weist das erfindungsgemässe Produkt einen höheren Reinheitsgrad auf und braucht darüberhinaus zur Abtrennung von Lösungsmittelresten nicht lyophilisiert zu werden, sondern kann nach gewöhnlichem Trocknen im Vakuum weiterverarbeitet werden. Nach Vermahlen der Kristalle lässt sich das kristalline Produkt ohne Einbusse an Stabilität in eine maschinell abfüllbare, d.h. fliessbare, Form überführen und kann in der gewünschten Menge ohne Schwierigkeiten zu lagerstabilen parenteral applizierbaren Arzneimittelzubereitungen verarbeitet werden.

Die erfindungsgemässe kristalline (5R,6S)-2-Aminomethyl-6-[(1R)-1-hydroxyäthyl]-2-penem-3-carbonsäure weist überraschende pharmakologische Eigenschaften auf. Beispielsweise ist sie in vitro gegen grampositive und gramnegative Kokken, z.B. Staphylococcus aureus, Streptococcus pyogenes, Streptococcus pneumoniae, Streptococcus faecalis, Neisseria meningitidis und Neisseria gonorrhoeae, gegen Enterobakterien, z.B. Escherichia coli, Proteus mirabilis und Klebsiella pneumoniae, gegen Haemophilus influenzae, Pseudomonas aeruginosa und Anaerobier, z.B. Bacteroides sp., und Clostridium sp. in minimalen Konzentrationen von ca. 0,05 bis ca. 8 µg/ml wirksam. In vivo, bei der systemischen Infektion der Maus, z.B. durch Staphylococcus aureus, Escherichia coli oder Streptococcus pyogenes, ergeben sich bei parenteraler, wie subkutaner, Applikation $ED_{50}$-Werte von ca. 0,3 bis ca. 30 mg/kg.

Kristalline (5R,6S)-2-Aminomethyl-6-[(1R)-1-hydroxyäthyl]-2-penem-3-carbonsäure (Verbindung A) weist im Vergleich zu der aus dem U.S.-Patent Nr. 4,272,437 vorbekannten amorphen, racemischen (1'R,5R,6S + 1'S,5S,6R)-2-Aminomethyl-6-(1'-hydroxyäthyl)-2-penem-3-carbonsäure (Verbindung B) in vitro die folgende überlegene Wirksamkeit auf:

Tabelle 1: Antibiotische Wirksamkeit der (5R,6S)-2-Aminomethyl-6-[(1R)-1-hydroxyäthyl]-2-penem-3-carbonsäure und der vorbekannten Vergleichs-Verbindung B in vitro

| Mikroorganismus | in vitro MIC (µg/ml) Verbindung A | Verbindung B |
|---|---|---|
| Staphylococcus aureus 10 B | 0.05 | 0.1 |
| Staphylococcus aureus 2999i+p+ | 0.05 | 0.2 |
| Staphylococcus aureus A 124 | 0.1 | 0.2 |
| Staphylococcus aureus Wood 46 | 0.05 | 0.1 |
| Streptococcus pyogenes Aronson 1129 | 0.2 | 0.5 |
| Streptococcus pneumoniae III/84 | 0.1 | 0.2 |
| Neisseria meningitidis 1316 | 0.5 | 1 |
| Neisseria gonorrhoeae 1317/4 | 0.5 | 1 |
| Haemophilus influenzae NCTC 4560 | 1 | 2 |
| Escherichia coli 205 | 2 | 8 |
| Escherichia coli 205 R + TEM | 4 | 8 |
| Escherichia coli 16 | 4 | 8 |
| Escherichia coli 2018 | 2 | 4 |
| Escherichia coli UB 1005 | 4 | 16 |
| Escherichia coli DC 2 | 8 | 16 |
| Escherichia coli B-1385 | 4 | 8 |
| Klebsiella pneumoniae 327 | 2 | 4 |
| Serratia marcescens 344 | 4 | 8 |
| Enterobacter cloacae P 99 | 4 | 8 |
| Enterobacter cloacae ATCC 13047 | 4 | 16 |
| Proteus mirabilis 774 | 1 | 4 |
| Proteus mirabilis 1219 | 2 | 8 |
| Proteus rettgeri 856 | 0.5 | 1 |
| Proteus morganii 2359 | 0.5 | 2 |
| Proteus morganii 1518 | 2 | 4 |
| Pseudomonas aeruginosa ATCC 12055 | 0.05 | 0.1 |
| Pseudomonas aeruginosa K 799/61 | 0.1 | 0.2 |
| Pseudomonas aeruginosa 143738R | 0.5 | 2 |
| Clostridium perfringens | 2 | 4 |
| Bacteroides fragilis L 01 | 0.5 | 1 |

Gegenüber dem entsprechenden vorbekannten amorphen Racemat (Verbindung B) weist die erfindungsgemässe kristalline Verbindung A bei allen getesteten Stämmen eine um den Faktor 2-4 höhere Aktivität auf.

Die Verbindungen A und B weisen gegenüber dem Enzym Dehydropeptidase aus menschlichen Nieren die folgenden Stabilitäten (ausgedrückt in Halbwertzeiten $t_{1/2}$) auf:

| | $t_{1/2}$ (Stunden) |
|---|---|
| Verbindung A | 6,75 |
| Verbindung B | 2,20 |

Gegenüber dem vorbeschriebenen amorphen Racemat (Verbindung B) besitzt die erfindungsgemässe kristalline Verbindung A überraschenderweise eine erheblich grössere Halbwertzeit bei der Einwirkung von renaler Dehydropeptidase.

Kristalline (5R,6S)-2-Aminomethyl-6-[(1R)-1-hydroxyäthyl]-2-penem-3-carbonsäure kann daher als parenteral applizierbares antibakterielles Antibiotikum, z.B. in Form von entsprechenden pharmazeutischen Präparaten, zur Behandlung von Infektionen Verwendung finden.

Die Erfindung betrifft ebenfalls ein Verfahren zur Herstellung von kristalliner (5R,6S)-2-Aminomethyl-6-[(1R)-1-hydroxyäthyl]-2-penem-3-carbonsäure. Ueberraschenderweise wurde gefunden, dass man das kristalline Produkt erhalten kann, indem man (5R,6S)-2-Aminomethyl-6-[(1R)-1-hydroxyäthyl]-2-penem-3-carbonsäure aus einer übersättigten Lösung in einem wasserhaltigen organischen Lösungsmittel zur Kristallisation bringt, das Produkt isoliert und trocknet.

Unter einer Lösung der (5R,6S)-2-Aminomethyl-6-[(1R)-1-hydroxyäthyl]-2-penem-3-carbonsäure in einem wasserhaltigen organischen Lösungsmittel ist beispielsweise eine Lösung in einer Lösungsmittelmischung bestehend aus Wasser und einem geeigneten mit Wasser mischbaren

organischen Lösungsmittel zu verstehen. Als organische Lösungsmittel kommen insbesondere Alkohole, wie niedere Alkanole mit 1 bis 4 Kohlenstoffatomen, z.B. Methanol, Aethanol, n-Propanol, Isopropanol, n-Butanol, 2-Butanol und sek. Butanol, Glykole mit 1 bis 4 Kohlenstoffatomen, z.B. Aethylenglykol, und deren Mono- und Di-($C_1$-$C_2$)-alkyläther, z.B. Aethylenglykolmonomethyläther, Aethylenglykoldimethyläther und Aethylenglykolmonoäthyläther, in Frage. Eine aus Wasser und dem mit Wasser mischbaren organischen Lösungsmittel bestehende Lösungsmittelmischung hat z.B. einen Wassergehalt von 2 bis 20 %, insbesondere von 2 bis 10 %. Bevorzugt sind wässrig-alkoholische Lösungen, wie insbesondere wässrig-äthanolische, wässrig-propanolische und wässrig-butanolische, wie wässrig-2-butanolische Lösungen, mit einem Wassergehalt von 2 bis 10 %.

Eine übersättigte Lösung kann hergestellt werden, indem man irgendeine Form der (5R,6S)-2-Aminomethyl-6-[(1R)-1-hydroxyäthyl]-2-penem-3-carbonsäure, z.B. die amorphe Form oder Mischungen der amorphen und der kristallinen Form, in einer Mischung von Wasser und einem der oben genannten organischen Lösungsmittel bei Raumtemperatur oder bei erhöhter Temperatur, z.B. bis zum Siedepunkt des verwendeten Lösungsmittels, bevorzugt aber bis maximal 50°C, unter Vermeidung oder Entfernung vorhandener Kristallisationskeime löst und die erhaltene reine Lösung in den Zustand der Uebersättigung, wie unten beschrieben, bringt. Dazu wird die Verbindung vorzugsweise in Wasser gelöst und dann das organische Lösungsmittel zugegeben. Man kann aber auch eine fertige Mischung aus Wasser und dem organischen Lösungsmittel verwenden und die (5R,6S)-2-Aminomethyl-6-[(1R)-1-hydroxyäthyl]-2-penem-3-carbonsäure gegebenenfalls unter Erwärmen, z.B. bis zum Siedepunkt der Mischung, bevorzugt aber bis maximal 50°C, darin lösen. Im Verlauf der Kristallisation oder vorher kann ein Teil des Lösungsmittels oder Lösungsmittelgemisches abdestilliert werden, um den Grad der Uebersättigung zu steigern.

Die übersättigte Lösung kann z.B. erhalten werden, indem man eine gesättigte oder nahezu gesättigte, gegebenenfalls warme, d.h. ca. 20° bis ca. 50°C warme Lösung der Verbindung, die von jeglichen

Kristallisationskeimen frei ist, abkühlt, bis Uebersättigung eintritt, z.B. auf etwa 0° bis etwa 20°C, oder einen Teil des Lösungsmittels oder der Lösungsmittelmischung durch Destillation entfernt. Der Kühlvorgang wird vorzugsweise langsam unter Rühren durchgeführt.

Die Kristallbildung kann spontan erfolgen, beispielsweise an der Oberfläche des Reaktionsgefässes bzw. Rührgerätes, kann aber auch durch Animpfen, d.h. Einbringen von Impfkristallen, ausgelöst werden. Stehen keine Impfkristalle zur Verfügung, können diese, vorteilhaft in einem aliquoten Teil der Lösung, in üblicher Weise, beispielsweise durch heftiges Schütteln, Einbringen von Glasstaub oder Ritzen der Gefässwand, hergestellt werden. Man kann aber auch das gesamte Lösungsvolumen geringfügig, d.h. um einige, z.B. 2 bis 5°C, unterkühlen, um die spontane Kristallbildung zu fördern, und dann wieder auf die Ausgangstemperatur erwärmen.

Man kann die kristalline Verbindung auch herstellen, indem man die amorphe Verbindung in einem Lösungsmittelgemisch, indem sie nur wenig löslich ist, wie in einem der genannten organischen Lösungsmittel mit geringem Wasseranteil, wie in einem der genannten niederen Alkanole mit 2 bis 10 % Wassergehalt, z.B. in 90 %igem bis 98 %igem Aethanol, bei Raumtemperatur oder geringfügig erhöhter oder erniedrigter Temperatur, z.B. bei 15° bis 25°C, unter Rühren digeriert. Dabei wandelt sich die amorphe Form in die kristalline Form um. Die Umwandlung ist in der Regel nach etwa 20 bis 30 Minuten abgeschlossen, so dass das Digerieren nach dieser Zeit, spätestens aber nach ca. 60 Minuten abgebrochen werden kann.

Die gebildete neue kristalline Verbindung kann mit Hilfe einer beliebigen für die Trennung binärer fest/flüssiger Systeme zur Verfügung stehenden Methode isoliert und gesammelt werden, beispielsweise durch Filtration, Druckfiltration ("Abnutschen"), Zentrifugieren oder Dekantieren. Zur Entfernung von in verbleibenden Mutterlaugeresten

enthaltenen Verunreinigungen kann mit Wasser oder dem reinen zur Kristallisation verwendeten Lösungsmittelgemisch nachgewaschen werden.

Die Trocknung wird bei normaler oder leicht erhöhter Temperatur, beispielsweise im Temperaturbereich von etwa 15°C bis etwa 40°C, vorzugsweise bei etwa 20° bis etwa 25°C (Raumtemperatur), durchgeführt und bis zu annähernder Gewichtskonstanz fortgesetzt. Zur Beschleunigung der Trocknung kann unter vermindertem Druck gearbeitet werden, wobei z.B. sogenanntes Wasserstrahlvakuum (etwa 650 bis etwa 3300 Pa) oder Hochvakuum (etwa 5 bis etwa 100 Pa) angewendet werden kann.

Das Ausgangsmaterial, amorphe (5R,6S)-2-Aminomethyl-6-[(1R)-1-hydroxyäthyl]-2-penem-3-carbonsäure, ist noch nicht bekannt und kann hergestellt werden, indem man in einer Verbindung der Formel

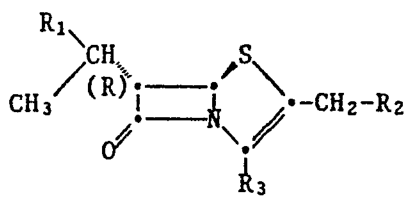

(I),

worin $R_1$ eine geschützte Hydroxylgruppe ist, $R_2$ eine geschützte Aminogruppe ist und $R_3$ eine geschützte Carboxylgruppe darstellt, die geschützten funktionellen Gruppen in die freien funktionellen Gruppen überführt.

Eine geschützte Hydroxylgruppe $R_1$ ist beispielsweise Tri-($C_1$-$C_4$)-alkylsilyloxy, z.B. Trimethylsilyloxy oder tert.Butyldimethylsilyloxy, Halogen-($C_1$-$C_4$)-alkoxycarbonyloxy, worin Halogen z.B. Chlor oder Brom ist, z.B. 2-Brom- oder 2,2,2-Trichloräthoxycarbonyloxy, oder gegebenenfalls durch Halogen, wie Chlor oder Brom, substituiertes ($C_1$-$C_4$)-Niederalkenyloxycarbonyloxy, z.B. Allyloxycarbonyloxy oder 2-Chlorallyloxycarbonyloxy.

Eine geschützte Aminogruppe $R_2$ ist z.B. Azido, Phthalimido, gegebenenfalls durch Halogen, wie Chlor oder Brom, substituiertes ($C_1$-$C_4$)-Niederalkenyloxycarbonylamino, z.B. Allyloxycarbonylamino oder 2-Chlorallyloxycarbonylamino, oder gegebenenfalls durch Nitro substituiertes Benzyloxycarbonylamino, z.B. p-Nitrobenzyloxycarbonylamino.

Eine geschützte Carboxylgruppe $R_3$ ist z.B. gegebenenfalls durch Nitro substituiertes Benzyloxycarbonyl, z.B. p-Nitrobenzyloxycarbonyl, gegebenenfalls durch Halogen, wie Chlor oder Brom, substituiertes ($C_1$-$C_4$)-Niederalkenyloxycarbonyl, z.B. Allyloxycarbonyl oder 2-Chlorallyloxycarbonyl, oder in 2-Stellung durch Cyano oder Tri-($C_1$-$C_4$)-niederalkylsilyl, z.B. Di-n-butylmethyl oder Trimethylsilyl, substituiertes Aethoxycarbonyl.

Die Schutzgruppen können stufenweise oder gleichzeitig abgespalten werden. Bevorzugt ist die Ausführungsform, bei der alle drei Schutzgruppen in einem Schritt, d.h. simultan, abgespalten werden.

Die Abspaltung der Schutzgruppen erfolgt nach an sich bekannten Verfahren. So kann eine durch Tri-($C_1$-$C_4$)-alkylsilyl geschützte Hydroxygruppe $R_1$ und in 2-Stellung durch Tri-($C_1$-$C_4$)-niederalkylsilyl substituiertes Aethoxycarbonyl $R_3$ z.B. durch Behandeln mit einem Fluoridanionen liefernden Salz der Fluorwasserstoffsäure, wie mit einem Alkalimetallfluorid, z.B. Natriumfluorid, in Anwesenheit eines makrocyclischen Polyäthers ("Kronenäther") oder mit dem Fluorid einer organischen quaternären Base, wie Tetra-($C_1$-$C_4$)-alkylammoniumfluorid, z.B. Tetraäthylammoniumfluorid, in die freie Hydroxygruppe $R_1$ bzw. in die freie Carboxylgruppe $R_3$ überführt werden. Halogen-($C_1$-$C_4$)-alkoxycarbonyloxy $R_1$, Azido $R_2$, gegebenenfalls durch Nitro substituiertes Benzyloxycarbonylamino $R_2$ oder Benzyloxycarbonyl $R_3$ können durch Reduktion, beispielsweise durch katalytische Hydrierung mit Wasserstoff in Gegenwart eines geeigneten Hydrierkatalysators, z.B. Platinoxid oder Palladium, in freies Hydroxy $R_1$, freies Amino $R_2$ bzw. freies Carboxyl $R_3$ überführt werden. Die Ueberführung einer in Form einer Phthalimidogruppe geschützten Aminogruppe $R_2$ in freies Amino $R_2$ erfolgt beispiels-

weise durch Umsetzen mit Hydrazin. Eine in 2-Stellung durch Cyano substituierte Aethoxycarbonylgruppe $R_3$ kann z.B. durch Behandeln mit einem basischen Mittel, z.B. einem Alkalimetallhydroxid oder -carbonat, wie Natrium- oder Kaliumcarbonat, in freies Carboxyl $R_3$ überführt werden.

In einer bevorzugten Ausführungsform des Verfahrens steht $R_1$ für ($C_1$-$C_4$)-Niederalkenyloxycarbonyloxy, insbesondere Allyloxycarbonyloxy, $R_2$ für ($C_1$-$C_4$)-Niederalkenyloxycarbonylamino, insbesondere Allyloxycarbonylamino, und $R_3$ für ($C_1$-$C_4$)-Niederalkenyloxycarbonyl, insbesondere Allyloxycarbonyl. Die Wahl der genannten bevorzugten, insbesondere Allyl-haltigen, Schutzgruppen ermöglicht die Freisetzung aller drei funktionellen Gruppen in einem Schritt.

Das Verfahren zur gleichzeitigen Ueberführung einer ($C_1$-$C_4$)-Niederalkenyloxycarbonyloxy-Gruppe $R_1$ in Hydroxy, einer ($C_1$-$C_4$)-Niederalkenyloxycarbonylamino-Gruppe $R_2$ in Amino und einer ($C_1$-$C_4$)-Niederalkenyloxycarbonyl-Gruppe $R_3$ in Carboxyl, wobei Niederalkenyl insbesondere für Allyl steht, ist dadurch gekennzeichnet, dass eine Verbindung der Formel I mit einem Niederalkenylgruppen-Akzeptor in Gegenwart von Tetrakis-triphenylphosphin-palladium und gegebenenfalls in Gegenwart von Triphenylphosphin umgesetzt wird.

Geeignete Akzeptoren für Niederalkenylgruppen, wie insbesondere die Allylgruppe, sind z.B. Amine, wie insbesondere sterisch gehinderte Amine, z.B. tert.Butylamin, ferner Tri-($C_1$-$C_4$)-niederalkylamine, z.B. Triäthylamin, Morpholin oder Thiomorpholin, aliphatische oder cycloaliphatische ß-Dicarbonylverbindungen, z.B. Acetylaceton, Acetessigsäureäthylester oder Dimedon, ferner auch ($C_2$-$C_4$)-Niederalkancarbonsäuren, z.B. Essigsäure oder Propionsäure. Bevorzugter Akzeptor ist Dimedon.

Die Reaktion wird mit 1,5 bis 10 Moläquivalenten des Niederalkenylgruppen-Akzeptors in Gegenwart von 2 bis 10 Mol-%, insbesondere 5 bis 8 Mol-% (bezogen auf die Ausgangsverbindung der Formel I), Tetrakis-triphenylphosphin-palladium-Katalysator, und gegebenenfalls

in Gegenwart von bis zu 50 Mol-% Triphenylphosphin in einem inerten Lösungsmittel, wie einem Aether, z.B. Dioxan oder insbesondere Tetrahydrofuran, einem Halogenkohlenwasserstoff, z.B. Methylenchlorid, einem niederen Alkanol, z.B. Aethanol, einem Ester, z.B. Aethylacetat, oder in einem Gemisch davon bei Raumtemperatur oder etwas erhöhter oder erniedrigter Temperatur, z.B. bei etwa 0° bis etwa 40°C, bevorzugt bei Raumtemperatur, erforderlichenfalls in einer Inertgasatmosphäre, wie in einer Stickstoff- oder Argonatmosphäre, durchgeführt. Dabei fällt die (5R,6S)-2-Aminomethyl-6-[(1R)-1-hydroxyäthyl]-2-penem-3-carbonsäure als amorpher Niederschlag aus der Reaktionsmischung aus.

Bei Verwendung von Dimedon als Niederalkenylgruppen-Akzeptor und Tetrahydrofuran als Lösungsmittel fällt die (5R,6S)-2-Aminomethyl-6-[(1R)-1-hydroxyäthyl]-2-penem-3-carbonsäure in überraschend hoher Reinheit aus (Reinheitsgrad grösser als 90 %), so dass das Produkt nach dem Entfärben mit Aktivkohle direkt dem Kristallisationsverfahren unterworfen werden kann, ohne dass weitere Reinigungsschritte, wie Säulenchromatographie und dergleichen, erforderlich sind.

Das Verfahren zur Herstellung von (5R,6S)-2-Aminomethyl-6-[(1R)-1-hydroxyäthyl]-2-penem-3-carbonsäure aus den Verbindungen der Formel I ist ebenfalls Gegenstand der vorliegenden Erfindung.

Die Ausgangsverbindungen der Formel I können nach an sich bekannten Verfahren, beispielsweise nach dem in der Europäischen Patentanmeldung Nr. 82113 oder in der Deutschen Offenlegungsschrift Nr. 3224055 beschriebenen Verfahren, hergestellt werden.

Die Erfindung betrifft weiterhin pharmazeutische Präparate, welche eine therapeutisch wirksame Menge der kristallinen (5R,6S)-2-Aminomethyl-6-[(1R)-1-hydroxyäthyl]-2-penem-3-carbonsäure zusammen oder im Gemisch mit anorganischen oder organischen, festen oder flüssigen, pharmazeutisch annehmbaren Trägerstoffen enthalten, die sich zur parenteralen, d.h. z.B. intramuskulären, intravenösen, subcutanen oder intraperitonealen, Verabreichung eignen.

Zur parenteralen Verabreichung eignen sich in erster Linie Infusionslösungen, vorzugsweise isotonische wässrige Lösungen oder Suspensionen, wobei diese z.B. aus Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventioneller Mischungs- und Lösungsverfahren, hergestellt. Das nach dem Vermahlen von kristalliner (5R,6S)-2-Aminomethyl-6-[(1R)-1-hydroxyäthyl]-2-penem-3-carbonsäure anfallende rieselfähige Pulver sowie gegebenenfalls zusätzliche Trägerstoffe wie Mannit können maschinell unter aseptischen Bedingungen auch direkt in der gewünschten Menge in Vials oder Ampullen abgefüllt werden. Die Präparate der vorliegenden Erfindung enthalten von etwa 0,1 % bis 100 %, Zubereitungen in Ampullen von etwa 50 % bis zu 100 % des Aktivstoffs.

Je nach Art der Infektion und individuellem Zustand des infizierten Organismus verwendet man tägliche parenterale Dosen von etwa 100 mg bis etwa 5 g des Wirkstoffs zur Behandlung von Warmblütern (Menschen und Tiere) von etwa 70 kg Gewicht.

Die Verwendung der erfindungsgemässen kristallinen Penem-Verbindung zur therapeutischen Behandlung des menschlichen und tierischen Körpers bildet ebenfalls einen Gegenstand der vorliegenden Erfindung.

Die folgenden Beispiele dienen zur Illustration der Erfindung. Temperaturen werden in Celsiusgraden angegeben.

Beispiel 1: 1-[(3S,4R)-3-[(1R)-1-Allyloxycarbonyloxyäthyl]-4-(allyloxycarbonylaminoacetylthio)-2-oxo-azetidin-1-yl]-2-triphenylphosphoranylidenessigsäureallylester.

0,385 g Silbersalz des 2-[(3S,4R)-3-[(1R)-1-Allyloxycarbonyloxyäthyl]-4-mercapto-2-oxo-azetidin-1-yl]-2-triphenylphosphoranylidenessigsäure-allylesters werden in 10 ml abs. Methylenchlorid mit 0,5 ml Pyridin und anschliessend bei 0° tropfenweise mit einem Gemisch aus 0,13 g Allyloxycarbonylaminoacetylchlorid und 10 ml abs. Methylenchlorid versetzt. Nach 30 Minuten Rühren wird der Feststoff über Hyflo abfilriert und das Filtrat wird mit wässriger $NaHCO_3$-Lösung und dann mit Sole gewaschen. Nach Trocknen über $Na_2SO_4$ wird im Vakuum eingeengt. Der Rückstand wird durch Chromatographie an Silicagel (Laufmittel Toluol/Aethylacetat 4:1) gereinigt. IR ($CH_2Cl_2$): 3440, 1750, 1740, 1700, 1620 $cm^{-1}$.

Das Ausgangsmaterial Allyloxycarbonylaminoacetylchlorid kann wie folgt hergestellt werden.

aa) Allyloxycarbonylaminoessigsäure

Zu einer Lösung von 7,51 g Glycin in 20 ml Wasser und 44 ml 5 N NaOH-Lösung wird bei 0° 12 ml Chlorameisensäureallylester tropfenweise zugegeben. Die Suspension wird 16 Stunden bei Raumtemperatur nachgerührt. Nach dem Entfernen des Unlöslichen durch Filtration wird das Filtrat mit 100 ml Wasser verdünnt und zweimal mit $CH_2Cl_2$ gewaschen. Die wässrige Phase wird mit HCl 4 N auf pH 2 gestellt und zweimal mit $CH_2Cl_2$ extrahiert. Die vereinten organischen Extrakte werden einmal mit Sole gewaschen, über $MgSO_4$ getrocknet und zu den weissen Kristallen der Titelverbindung eingedampft. IR in $CH_2Cl_2$: 3450; 1715 $cm^{-1}$.

0181571

ab) Allyloxycarbonylaminoacetylchlorid

3,18 g Allyloxycarbonylaminoessigsäure werden bei 0° mit 5,7 ml Thionylchlorid versetzt. Das Gemisch wird dann bei gleicher Temperatur 2 Stunden unter Schutzgas gerührt. Anschliessend wird mit absolutem Toluol verdünnt und am Rotationsverdampfer eingeengt.
IR ($CH_2Cl_2$): 3435; 1800; 1725 $cm^{-1}$.

Das Silbersalz des 2-[(3S,4R)-3-[(1R)-1-Allyloxycarbonyloxyäthyl]-4-mercapto-2-oxoazetidin-1-yl]-2-triphenylphosphoranylidenessigsäure-allylesters wird folgendermassen hergestellt:

ba) (2S,3R)-2-Brom-3-hydroxybuttersäure-p-methoxybenzylamid

Zu einer bei Raumtemperatur unter Argonatmosphäre gerührten Lösung von 5 g (2S,3R)-2-Brom-3-hydroxybuttersäure und 3,52 g p-Methoxy-benzylamin in 60 ml abs. THF werden innerhalb 20 Minuten 4,16 g 1-Hydroxybenztriazol und 5,63 g Dicyclohexylcarbodiimid in 60 ml THF zugetropft. Man rührt das Reaktionsgemisch 48 Stunden, filtriert den ausgefallenen Dicyclohexylharnstoff ab, wäscht diesen mehrmals mit THF und dampft das Filtrat ein. Das erhaltene Rohprodukt enthält Dicyclohexylharnstoff und Hydroxybenztriazol als Verunreinigung. Nach Chromatographieren des Gemisches an Silicagel (System: Toluol; Toluol/Aethylacetat 1:4) und Kristallisation der reinen Fraktionen aus Methylenchlorid/Diäthyläther wird die Titelverbindung vom F. 122-124° erhalten.
$[\alpha] = -7 \pm 1°$ (1,112 % in Chloroform).

bb) (2R,3R)-2,3-Epoxybuttersäure-p-methoxybenzylamid

Eine Lösung von 6,04 g (20 mM) (2S,3R)-2-Brom-3-hydroxybuttersäure-p-methoxybenzylamid in 150 ml Methylenchlorid wird mit 50 ml 50 %iger NaOH-Lösung un 456 mg (2 mM) Benzyltriäthylammoniumchlorid versetzt. Das zweiphasige Gemisch wird während 20 Stunden bei Raumtemperatur stark gerührt. Die organische Schicht wird abgetrennt und die wässrige Phase mit Methylenchlorid nachextrahiert. Die vereinigten Methylenchlorid-Lösungen werden getrocknet und eingedampft. Das anfallende Rohprodukt wird an 40-facher Gewichtsmenge Silicagel im System Methylenchlorid/Methanol (99:1) chromatographiert. Nach Kristallisation der reinen Fraktionen aus Methylenchlorid-Diäthyläther-Petrolether wird die Titelverbindung, F.: 75-76°, erhalten.

bc) (2R,3R)-2,3-Epoxybuttersäure-N-tert-butoxycarbonylmethyl-N-p-methoxybenzylamid

Zu einer bei 0° unter Argonatmosphäre gerührten Mischung von 550 mg Natriumhydrid-Dispersion (55-60 % in Oel) und 1,52 ml Bromessig-säuretert-butylester in 25 ml THF wird eine Lösung von 2,21 g (2R,3R)-2,3-Epoxybuttersäure-p-methoxybenzylamid in 100 ml THF zugetropft. Man erwärmt auf Raumtemperatur und rührt das Reaktionsgemisch 1 Stunde weiter (Kontrolle des Reaktionsablaufs mittels Dünnschichtchromatographie). Gesamtreaktionszeit: 90 Minuten. Die unlöslichen Anteile werden abfiltriert und mit THF gewaschen und die vereinigten Filtrate eingedampft. Das anfallende Rohprodukt reinigt man durch Chromatographieren an 150 g Silicagel (System: Toluol, Toluol/Aethylacetat 80:20). Durch Eindampfen der reinen Fraktionen erhält man die amorphe Titelverbindung. IR-Spektrum: Banden u.a. bei 1740, 1670, 1650, 1615, 1517, 1465, 1360 und 1035 $cm^{-1}$.

bd) (3S,4S)-1-(p-Methoxybenzyl)-3-[(1R)-1-hydroxyäthyl]-4-tert-butoxycarbonyl-2-azetidinon

9,23 g Tetrabutylammoniumfluorid-trihydrat werden mit 40 g Molekularsieb (Typ 4171/16 - bei 300° vorgetrocknet) in 80 ml THF 16 Stunden bei 5° stehen gelassen. Die Mischung wird auf 0° abgekühlt, mit einer Lösung von 2,8 g (2R,3R)-2,3-Epoxybuttersäure-N-tert-butoxycarbonylmethyl-Nmethoxybenzylamid in 20 ml THF versetzt und 2 Stunden bei 0-5° gerührt. Das Molekularsieb wird unter Nachwaschen mit THF abfiltriert und das Filtrat direkt auf eine in Toluol bereitete Säule mit 250 g Silicagel aufgetragen. Die eingedampften, mit Toluol/Aethylacetat (70:30-Gemisch) eluierten Fraktionen werden in Methylenchlorid aufgenommen, nacheinander zweimal mit 1N Schwefeläure, mit gesättigter, wässriger $NaHCO_3$-Lösung und mit Wasser gewaschen, getrocknet und eingedampft. Nach Kurzchromatographie an

Silicagel (Toluol, Toluol/Aethylacetat 60:40) und Kristallisation aus Methylenchlorid/Diäthyläther/Petroläther wird die reine Titelverbindung vom F. 85-87° erhalten.

be) (3S,4S)-1-(p-Methoxybenzyl)-3-[(1R)-1-allyloxycarbonyloxyäthyl]-4-tert-butoxycarbonyl-2-azetidinon

Eine Lösung von 2 g (3S,4S)-1-(p-Methoxybenzyl)-3-[(1R)-1-hydroxyäthyl)-4-tert-butoxycarbonyl-2-azetidinon in 24 ml Methylenchlorid wird bei 0° mit 24 ml 1N NaOH, 820 mg Tetrabutylammoniumhydrogensulfat und 1 ml Chlorameisensäureallylester versetzt und heftig gerührt. Nach 20 und 40 Minuten Reaktionszeit werden weitere Portionen (je 1 ml) Chlorameisensäureallylester zugegeben. Das Reaktionsgemisch wird mit Methylenchlorid verdünnt, die wässrige Phase abgetrennt und die organische Schicht nacheinander mit 5%-iger wässriger Zitronensäure und 8%-iger wässriger $NaHCO_3$-Lösung gewaschen, getrocknet und eingedampft. Nach chromatographischer Reinigung wird die reine, amorphe Titelverbindung. IR-Spektrum: Banden u.a. bei 1765, 1745(sh), 1615, 1593, 1515, 1160 und 1035 $cm^{-1}$.

bf) (3S,4S)-1-(p-Methoxybenzyl)-3-[(1R)-1-allyloxycarbonyloxyäthyl]-4-carbonsäure-2-azetidinon

1,6 g (3S,4S)-1-(p-Methoxybenzyl)-3-[(1R)-1-allyoxycarbonyloxyäthyl]-4-tert-butoxycarbonyl-2-azetidinon werden bei 0° in 10 ml Trifluoressigsäure gelöst. Nach einer Stunde Reaktionszeit bei Raumtemperatur wird das Reaktionsgemisch im Hochvakuum eingedampft und die erhältliche Titelverbindung ohne Reinigung weiterverarbeitet. [α]= +85 ± 1° (1,0 % in Chloroform).

bg) (3R,4R)-1-(p-Methoxybenzyl)-3-[(1R)-1-allyloxycarbonyloxyäthyl]-4-acetoxy-2-azetidinon

Eine unter Argonatmosphäre bei Raumtemperatur gerührte Lösung von 1,4 g (3S,4S)-1-(p-Methoxybenzyl)-3-[(1R)-1-allyloxycarbonyloxyäthyl]-4-carbonsäure-2-azetidinon in einem Gemisch aus 45 ml THF und 6,6 ml Dimethylformamid wird mit 1,6 g Blei-(IV)-acetat (ca.10 % Essigsäure-Gehalt) versetzt und ca. 1 Stunde bis zur vollständigen Umsetzung des Substrats gerührt. Ueberschüssiges Oxidationsmittel

wird durch Zugabe von 0,5 ml Aethylenglykol zersetzt (10 Min Raumtemperatur). Das Reaktionsgemisch wird von ausgefallenem Blei-(II)-acetat abfiltriert, der Filtrierrückstand mit THF ausgewaschen und das Filtrat eingedampft. Der erhaltene ölige Rückstand wird in Methylenchlorid aufgenommen, nacheinander je zweimal mit gesättigter, wässriger $NaHCO_3$-Lösung, Wasser und ges. NaCl-Lösung gewaschen, getrocknet und eingedampft. Durch Chromatographieren des Rückstands an Silicagel (Toluol; Toluol/Aethylacetat 90:10) wird die reine Titelverbindung [α]= +90° ± 1° (1,0 % in Chloroform) erhalten.

bh) <u>(3R,4R)-3-[(1R)-1-Allyloxycarbonyloxyäthyl]-4-acetoxy-2-azetidinon</u>

Eine Lösung von 900 mg (1,18 mMol) (3R,4R)-1-(p-Methoxybenzyl)-3-[1R)-1-allyloxycarbonyloxyäthyl]-4-acetoxy-2-azetidinon in 30 ml Acetonitril wird bei 10° mit einer Lösung von 5,37 g Cer-(IV)-ammoniumnitrat in 15 ml Wasser versetzt und zwei Stunden bei Raumtemperatur gerührt. Nach Extraktion mit Aethylacetat, Waschen mit gesättigter $NaHCO_3$-Lösung, Trocknen der organischen Phase über Natriumsulfat und Eindampfen unter vermindertem Druck erhält man die rohe Titelverbindung, welche man chromatographisch an Silicagel mit Toluol-Aethylacetat (4:1 und 1:1) reinigt. [α]= +84 ± 1° (1,0 % in Chloroform).

bi) <u>(3S,4R)-3-[(1R)-1-Allyloxycarbonyloxyäthyl]-4-triphenylmethylthio-azetidin-2-on</u>

892 mg Triphenylmethylmercaptan werden in 5 ml Methanol bei 0° suspendiert und über 10 Minuten portionsweise mit insgesamt 0,16 g einer 50%igen Natriumhydrid-Suspension in Oel versetzt. Anschliessend wird eine Lösung von 0,62 g (3R,4R)-3-[(1R)-1-Allyloxycarbonyloxyäthyl]-4-acetoxy-azetidin-2-on in 7 ml Aceton und 5 ml Wasser über 15 Minuten zugetropft. Nach 1 Stunde Rühren bei 0° und drei weiteren Stunden bei Raumtemperatur wird das Reaktionsgemisch am Rotationsverdampfer eingeengt und mit 20 ml Methylenchlorid extrahiert. Die organische Phase wird mit Sole gewaschen und über $MgSO_4$ getrocknet. Nach Einengen wird die rohe Titelverbindung durch Chromatographie auf Silicagel (Laufmittel Toluol-Aethylacetat 19:1) gereinigt.

DC (Toluol-Aethylacetat 4:1) $R_f$ = 0,3, IR ($CH_2Cl_2$) 3400; 1770 1745 $cm^{-1}$.

bj) 2-[(3S,4R)-3-[(1R)-1-Allyloxycarbonyloxyäthyl]-4-triphenylmethylthio-2-oxo-azetidin-1-yl]-2-hydroxyessigsäure-allylester

0,82 g (3S,4R)-3-[(1R)-1-Allyloxycarbonyloxyäthyl]-4-triphenylmethylthio-azetidin-2-on und 0,416 g Glyoxylsäureallylester-äthylhemiacetal in 10 ml abs. Toluol werden mit 4 g Molekularsieb (4Å) versetzt und während 61 Stunden bei 60° gerührt. Nach Abfiltrieren und Einengen am Rotationsverdampfer unter vermindertem Druck wird die Titelverbindung erhalten. DC (Silicagel/Toluol/Aethylacetat 4:1), $R_f$ = 0,1. IR ($CH_2Cl_2$): 3510; 1770; 1745 $cm^{-1}$.

bk) 2-[(3S,4R)-3-[(1R)-1-Allyloxycarbonyloxyäthyl]-4-triphenylmethylthio-2-oxo-azetidin-1-yl]-2-triphenylphosphoranyliden-essigsäure-allylester

Zu einer Lösung von 1 g 2-[(3S,4R)-3-[(1R)-1-Allyloxycarbonyloxyäthyl]-4-triphenylmethylthio-2-oxo-azetidin-1-yl]-2-hydroxyessigsäure-allylester in 10 ml Tetrahydrofuran werden unter Rühren bei 0° nacheinander 0,182 ml Thionylchlorid und 0,206 ml Pyridin innert 5 Minuten zugegeben. Die weisse Suspension wird 30 Minuten bei 0° gerührt und über Hyflo filtriert. Nach Waschen des Rückstands mit Toluol wird am Rotationsverdampfer eingeengt. Der Rückstand wird in 10 ml Dioxan gelöst, mit 0,624 g Triphenylphosphin und 0,257 ml Lutidin versetzt und während 46 Stunden bei 80° Badtemperatur gerührt. Das Gemisch wird über Hyflo filtriert und der Rückstand mit Toluol nachgewaschen. Die vereinten Filtrate werden eingedampft und die Chromatographie des Rückstandes an Silicagel ergibt das reine Produkt (Laufmittel Toluol/Aethylacetat 19:1 bis 4:1), DC (Silicagel; Toluol/Aethylacetat 4:1), $R_f$ = 0,24, IR ($CH_2Cl_2$) 1745; 1620 $cm^{-1}$.

b1) Silbersalz des 2-[(3S,4R)-3-[(1R)-1-Allyloxycarbonyloxyäthyl]-4-triphenylmethylthio-2-oxo-azetidin-1-yl]-2-triphenylphosphoranyliden-essigsäureallylesters

0,46 g 2-[(3S,4R)-3-[(1R)-1-Allyloxycarbonyloxyäthyl]-4-triphenylmethylthio-2-oxo-azetidin-1-yl]-2-triphenylphosphoranylidenessigsäureallylester werden in 6 ml Diäthyläther vorgelegt und bei Raumtemperatur mit 4,4 ml einer 0,5M wässrigen Silbernitrat-Lösung versetzt. Danach gibt man 0,077 ml Triäthylamin dazu und rührt das Reaktionsgemisch während 30 Minuten nach. Der Feststoff wird abgenutscht und gut mit Wasser und Diäthyläther gewaschen. Der Rückstand wird nochmals in 300 ml Wasser und 300 ml Diäthyläther aufgeschlämmt, gerührt und dann abfiltriert. Nach erneutem Waschen mit Diäthyläther wird der Feststoff am Hochvakuum getrocknet. IR ($CH_2Cl_2$) 1760; 1745; 1630 $cm^{-1}$.

Beispiel 2: (5R,6S)-2-Allyloxycarbonylaminomethyl-6-[(1R)-1-allyloxycarbonyloxyäthyl]-2-penem-3-carbonsäureallylester

Eine Lösung von 2,42 g 2-[(3S,4R)-3-[(1R)-1-Allyloxycarbonyloxyäthyl]-4-(allyloxycarbonylaminoacetylthio)-2-oxo-azetidin-1-yl]-2-triphenylphosphoranylidenessigsäure-allylester in 300 ml abs. Toluol wird unter Argonatmosphäre 24 Stunden bei Rückfluss-Temperatur gerührt. Dann wird das Lösungsmittel eingedampft und das Rohprodukt durch Chromatographie an Silicagel gereinigt. (Laufmittel Toluol-Aethylacetat 9:1). IR ($CH_2Cl_2$): 3435; 1790; 1740; 1720; 1580 $cm^{-1}$.

Beispiel 3: Amorphe (5R,6S)-2-Aminomethyl-6-[(1R)-1-hydroxyäthyl]-2-penem-3-carbonsäure

Variante A:

Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester, 308 mg (2,2 mMol) Dimedon und 30 mg (0,11 mMol) Triphenylphosphin in 4 ml Tetrahydrofuran wird während 5 Minuten mit Argon gespült. Dann werden bei Raumtemperatur 22 mg (0,019 mMol) Tetrakistriphenylphosphin-palladium zugegeben. Nach 5 Minuten beginnt ein Niederschlag auszufallen. Die Suspension wird insgesamt 1 Stunde bei

Raumtemperatur unter Argon gerührt. Das ausgefallene Produkt wird abfiltriert, mit Tetrahydrofuran, Aethylacetat und Hexan gewaschen und am Hochvakuum getrocknet. DC ($H_2O$, OPTI $UPC_{12}$), $R_f$ = 0,48.

Variante B:

Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester, 0,23 ml (2,2 mMol) Acetylaceton und 30 mg (0,11 mMol) Triphenylphosphin in 2 ml Tetrahydrofuran wird während 5 Minuten mit Argon gespült. Dann werden bei Raumtemperatur 20 mg (0,017 mMol) Tetrakis-triphenylphosphin-palladium zugegeben. Nach 5 Minuten fällt ein Niederschlag aus. Nach insgesamt 45 Minuten Reaktionszeit wird vom Niederschlag abfiltriert, dieser mit Tetrahydrofuran und Diäthyläther gewaschen und am Hochvakuum getrocknet. DC ($H_2O$, OPTI $UPC_{12}$), $R_f$ = 0,48.

Variante C: Eine Lösung von 100 mg (0,22 mMol (5R,6S)-2-Allyloxycarbonylaminomethyl-6-[(1R)-1-allyloxycarbonyloxyäthyl]-2-penem-3-carbonsäureallylester, 123 mg (0,88 mMol) Dimedon und 30 mg (0,11 mMol) Triphenylphosphin in 2 ml Tetrahydrofuran wird während 5 Minuten mit Argon gespült. Dann werden bei Raumtemperatur 22 mg (0,019 mMol) Tetrakis-triphenylphosphin-palladium zugegeben. Nach 1 Stunde wird vom Niederschlag abfiltriert, dieser mit Tetrahydrofuran und Diäthyläther gewaschen und am Hochvakuum getrocknet. DC ($H_2O$, OPTI $UPC_{12}$), $R_f$ = 0,48.

Variante D: Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester und 123 mg (0,88 mMol) Dimedon in 2 ml Tetrahydrofuran wird während 5 Minuten mit Argon gespült. Dann werden bei Raumtemperatur 22 mg (0,019 mMol) Tetrakis-triphenylphosphin-palladium zugegeben. Nach 1 Stunde wird vom Niederschlag abfiltriert, dieser mit Tetrahydrofuran und Diäthyläther gewaschen und am Hochvakuum getrocknet. DC ($H_2O$, OPTI $UPC_{12}$), $R_f$ = 0,48.

Variante E: Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester und 123 mg (0,88 mMol) Dimedon in 2 ml Aethylacetat wird während 5 Minuten mit Argon gespült. Dann werden bei Raumtemperatur 22 mg (0,019 mMol) Tetrakis-triphenylphosphinpalladium zugegeben. Nach 1 Stunde wird vom Niederschlag abfiltriert, dieser mit Aethylacetat und Diäthyläther gewaschen und am Hochvakuum getrocknet. DC ($H_2O$, OPTI $UPC_{12}$), $R_f$ = 0,48.

Variante F: Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester und 0,05 ml (0,88 mMol) Eisessig in 2 ml Tetrahydrofuran wird während 5 Minuten mit Argon gespült. Dann werden bei Raumtemperatur 22 mg (0,019 mMol) Tetrakis-triphenylphosphin-palladium zugegeben. Nach 1 Stunde wird vom Niederschlag abfiltriert, dieser mit Tetrahydrofuran und Diäthyläther gewaschen und am Hochvakuum getrocknet. DC ($H_2O$, OPTI $UPC_{12}$), $R_f$ = 0,48.

Variante G: Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester und 123 mg (0,88 mMol) Dimedon in 4 ml Methylenchlorid wird während 5 Minuten mit Argon gespült. Dann werden bei Raumtemperatur 22 mg (0,019 mMol) Tetrakis-triphenylphosphin-palladium zugegeben und bei Raumtemperatur weitergerührt. DC-Proben zeigen eine sehr langsame Reaktion. Nach 15 Stunden wird vom Niederschlag abfiltriert. Dieser wird mit Methylenchlorid gewaschen und am Hochvakuum getrocknet. DC ($H_2O$, OPTI $UPC_{12}$), $R_f$ = 0,48.

Variante H: Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester und 62 mg (0,44 mMol) Dimedon in 2 ml Tetrahydrofuran wird während 5 Minuten mit Argon gespült. Dann werden bei Raumtemperatur 22 mg (0,019 mMol) Tetrakis-triphenylphosphin-palladium zugegeben. Nach 1 Stunde wird vom Niederschlag abfiltriert, dieser mit Tetrahydrofuran und Aethylacetat gewaschen und am Hochvakuum getrocknet. DC ($H_2O$, OPTI $UPC_{12}$), $R_f$ = 0,48.

Variante I: Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester und 62 mg (0,44 mMol) Dimedon in 2 ml Tetrahydrofuran wird während 5 Minuten mit Argon gespült. Dann werden bei Raumtemperatur 5 mg (0,0043 mMol) Tetrakis-triphenylphosphin-palladium zugegeben. Nach 1 Stunde wird vom Niederschlag abfiltriert, dieser mit Tetrahydrofuran und Diäthyläther gewaschen und am Hochvakuum getrocknet. DC ($H_2O$, OPTI $UPC_{12}$), $R_f$ = 0,48.

Variante J: Eine Lösung von 100 mg (0,22 mMol) (5R,6S)-2-Allyloxycarbonylaminomethyl-6-[(1R)-1-allyloxycarbonyloxyäthyl]-2-penem-3-carbonsäureallylester und 123 mg (0,88 mMol) Dimedon in 2 ml Aethanol wird während 5 Minuten mit Argon gespült. Dann werden bei Raumtemperatur 10 mg (0,0086 mMol) Tetrakis-triphenylphosphin-palladium zugegeben. Nach 7 Stunden wird vom Niederschlag abfiltriert, dieser mit Tetrahydrofuran und Diäthyläther gewaschen und am Hochvakuum getrocknet. DC ($H_2O$, OPTI $UPC_{12}$), $R_f$ = 0,48.

Variante K: Eine Lösung von 11,3 g (25 mMol) (5R,6S)-2-Allyloxycarbonylaminomethyl-6-[(1R)-1-allyloxycarbonyloxyäthyl]-2-penem-3-carbonsäureallylester und 7,7 g (55 mMol) Dimedon in 220 ml Tetrahydrofuran wird während 10 Minuten mit Argon gespült und anschliessend mit 1,1 g (0,952 mMol) Tetrakis-triphenylphosphin-palladium versetzt. Die Lösung erwärmt sich leicht (ca. 30°C) und nach ca. 5 Minuten beginnt ein Niederschlag auszufallen. Nach 1 Stunde gibt man 0,2 ml (2,1 mMol) Methallylchlorid zu und lässt während 15 Minuten weiterrühren. Dann wird vom Niederschlag abfiltriert, dieser wird mit Tetrahydrofuran und Aethylacetat gewaschen und der feste Rückstand am Hochvakuum getrocknet. Der Rückstand ist amorph. DC ($H_2O$, OPTI $UPC_{12}$), $R_f$ = 0,48.

Beispiel 4: Herstellung von kristalliner (5R,6S)-2-Aminomethyl-6-[(1R)-1-hydroxyäthyl]-2-penem-3-carbonsäure

Variante A: (wasserhaltiges Aethanol)
20,4 g amorphe (5R,6S)-2-Aminomethyl-6-[(1R)-1-hydroxyäthyl]-2-penem-3-carbonsäure (siehe Beispiel 3) werden in 300 ml doppelt destilliertem Wasser bei 30° gelöst. Die wässerige Lösung wird auf 5° abgekühlt, mit 1 g Aktivkohle versetzt, 15 Minuten gerührt und anschliessend klarfiltriert. Das klare Filtrat wird am Hochvakuum vollständig eingeengt und der Rückstand in 190 ml Aethanol (96 %ig) aufgeschlemmt. Die Suspension wird bei Raumtemperatur während 30 Minuten stark gerührt. Die weissen Kristalle (Nadeln) werden abfiltriert und mit Aethanol (96 %ig) gewaschen. Das Produkt wird während 16h bei 20° am Hochvakuum (13 Pa) getrocknet.

Schmp. 165°C (Zers.); DC ($H_2O$, OPTI $UPC_{12}$) $R_f$ = 0,48; $\alpha_D^{20}$ (0,64 % in $H_2O$) +161,9° ± 1,6°; UV ($H_2O$) $\lambda_{max}$ 311 nm (ε 5100); IR (DMSO-$d_6$): 3431, 2970, 1774, 1628, 1574 $cm^{-1}$; $^1$H-NMR (360 MHz, $D_2O$): δ = 1,35 (d, $CH_3$), 4,02 (dd, C$\underline{H}$-CO), 4,07 (AB, $CH_2$), 4,28 (m, C$\underline{H}$-$CH_3$), 5,74 ppm (d, C$\underline{H}$-N).

$C_9H_{12}N_2O_4S \cdot 0,79\ H_2O$(Molekulargewicht 258,5)

| | berechnet | gefunden |
|---|---|---|
| C | 41,81 | 42,33 |
| H | 5,32 | 5,29 |
| N | 10,83 | 10,76 |
| S | 12,40 | 12,52 |
| $H_2O$ | 5,52 | 5,52 |

Röntgenpulverdiagramm der kristallinen (5R,6S)-2-Aminomethyl-6-[(1R)-1-hydroxythyl]-2-penem-3-carbonsäure

Zur Bestimmung der Netzebenenabstände (d-Werte) wurde das Beugungsbild auf Film registriert. Für die Aufnahme wurde eine Guinier-Kamera (Enraf-Nonius FR 552) mit Kupfer-$K_{\alpha 1}$-Strahlung (Wellenlänge = 1,54050 A) benutzt. Als Eichsubstanz wurde Quarz verwendet, dessen d-Werte aus a = 4,913 A und c = 5,405 A (PDF 5-490) berechnet wurden.

In der folgenden Tabelle sind die d-Werte der stärksten Linien mit d-Werten über 3,0 Angström angegeben, zusammen mit den von Auge geschätzten relativen Linienintensitäten.

| d-Werte (Angstroem) | relative Intensität |
|---|---|
| 10.9 | sehr stark |
| 10.0 | mittel |
| 9.7 | mittel |
| 7.9 | stark |
| 7.3 | stark |
| 7.0 | stark |
| 6.7 | schwach |
| 6.3 | sehr stark |
| 5.90 | sehr schwach |
| 5.85 | sehr schwach |
| 5.60 | sehr schwach |
| 5.53 | sehr schwach |
| 5.44 | sehr schwach |
| 5.33 | sehr schwach |
| 5.01 | sehr schwach |
| 4.93 | schwach |
| 4.64 | sehr schwach |
| 4.58 | sehr schwach |
| 4.53 | stark |
| 4.42 | sehr schwach |
| 4.34 | sehr stark |
| 4.28 | sehr schwach |
| 4.23 | sehr schwach |
| 4.10 | stark |
| 4.05 | sehr schwach |
| 3.99 | mittel |
| 3.91 | schwach |
| 3.82 | mittel |
| 3.78 | mittel |
| 3.73 | stark |
| 3.65 | mittel |
| 3.59 | stark |
| 3.54 | mittel |

| d-Werte (Angstroem) | relative Intensität |
| --- | --- |
| 3.42 | stark |
| 3.36 | mittel |
| 3.29 | stark |
| 3.20 | mittel |
| 3.13 | mittel |
| 3.11 | stark |
| 3.03 | mittel |
| 3.00 | mittel |

Variante B (wasserhaltiges n-Propanol)

8,2 g amorphe (5R,6S)-2-Aminomethyl-6-[(1R)-1-hydroxyäthyl]-2-penem-3-carbonsäure werden in 120 ml doppelt destilliertem Wasser bei 30° gelöst. Die wässerige Lösung wird auf 5° abgekühlt, mit 0,5 g Aktivkohle versetzt, 15 Minuten gerührt und anschliessend klarfiltriert. Das klare Filtrat wird am Hochvakuum vollständig eingeengt und der Rückstand in 80 ml n-Propanol (92%ig) aufgeschlemmt. Die Suspension wird bei Raumtemperatur während 30 Minuten stark gerührt. Die weissen Nadeln werden abfiltriert und mit n-Propanol (92%ig) gewaschen. Das Produkt wird während 15h bei 20° am Hochvakuum (14 Pa) getrocknet.

Schmp. 165° (Zers.); DC ($H_2O$, OPTI $UPC_{12}$) $R_f$ = 0,48.

$C_9H_{12}N_2O_4S \cdot 0,28\ H_2O$ (Molekulargewicht 249,4)

| | berechnet | gefunden |
| --- | --- | --- |
| C | 43,30 | 43,46 |
| H | 5,03 | 4,99 |
| N | 11,22 | 11,27 |
| S | 12,83 | 12,90 |
| $H_2O$ | 2,05 | 2,05 |

Abgesehen von geringfügigen Unterschieden in den geschätzten Linienintensitäten einiger Linien ist das Röntgenpulverdiagramm der Substanz mit dem unter Variante A beschriebenen Röntgenpulverdiagramm vollständig identisch.

Variante C (wasserhaltiges 2-Butanol)
14,9 g amorphe (5R,6S)-2-Aminomethyl-6-[(1R)-1-hydroxyäthyl]-2-penem-3-carbonsäure werden in 210 ml doppelt destilliertem Wasser bei 30° gelöst. Die wässrige Lösung wird auf 5° abgekühlt, mit 0,7 g Aktivkohle versetzt, 15 Minuten gerührt und anschliessend klarfiltriert. Das klare Filtrat wird am Hochvakuum vollständig eingeengt und der Rückstand in 140 ml 2-Butanol (90%ig) aufgeschlemmt. Die Suspension wird bei Raumtemperatur während 30 Minuten stark gerührt. Die weissen Kristalle (Nadeln) werden abfiltriert und mit 2-Butanol (90%ig) gewaschen. Das Produkt wird während 16h bei 20° am Hochvakuum (13 Pa) getrocknet.

Schmp. 165°C (Zers.); DC ($H_2O$, OPTI $UPC_{12}$) $R_f$ = 0,48.

$C_9H_{12}N_2O_4S \cdot 1,21\ H_2O$ (Molekulargewicht 266,06)

| | berechnet | gefunden |
|---|---|---|
| C | 40,63 | 40,78 |
| H | 5,49 | 5,53 |
| N | 10,53 | 10,43 |
| S | 12,05 | 11,53 |
| $H_2O$ | 8,19 | 8,19 |

Abgesehen von geringfügigen Unterschieden in den geschätzten Linienintensitäten einiger Linien ist das Röntgenpulverdiagramm der Substanz mit dem unter Variante A beschriebenen Röntgenpulverdiagramm vollständig identisch.

Beispiel 5: Bestimmung der thermischen Stabilität
Zur Bestimmung der thermischen Stabilität werden Proben der kristallinen (5R,6S)-2-Aminomethyl-6-[(1R)-1-hydroxyäthyl]-2-penem-3-carbonsäure und der aus der Deutschen Offenlegungsschrift Nr. 2 950 898 vorbekannten amorphen racemischen (5R,6S,1'R und 5S,6R,1'S)-2-Aminomethyl-6-(1'-hydroxyäthyl)-2-penem-3-carbonsäure verwendet.

Proben à 20 mg werden in Glasröhrchen eingewogen. Die Glasröhrchen werden mit einem PVC-Stopfen fest verschlossen und in einem thermostatierten Oelbad auf 50° erwärmt. In regelmässigen Abständen werden einzelne Glasröhrchen dem Oelbad entnommen, die Proben in 100 ml bidestilliertem Wasser aufgelöst und der Gehalt an Penem mittels HPLC-Analyse [UV-Detektor: Kratos, 305 nm; Einspritzautomat: Wisp; 20 µl Eingabe; Integrator: Shimazu, C-R3A; Pumpe: Altex, 2,0 ml/Min; Säule: Knauer, 25 cm x 4 mm, $C_{18}$/10 µm; Laufmittel: Acetonitril/Wasser 2:98, pH5] bestimmt.

Die folgenden Werte wurden erhalten (Probe A: kristalline (5R,6S)-2-Aminomethyl-6-[(1R)-1-hydroxyäthyl]-2-penem-3-carbonsäure; Probe B: amorphe (5R,6S,1'R und 5S,6R,1'S)-2-Aminomethyl-6-(1'-hydroxyäthyl)-2-penem-3-carbonsäure):

| Tag | Gehalt an Penem (%) Probe A | Probe B |
|---|---|---|
| 0 | ca. 100 | ca. 100 |
| 1,8 | 99 | 84 |
| 3 | 98 | 78 |
| 4,5 | 97 | 73 |
| 7 | 95 | 67 |
| 9 | 94 | 64 |
| 12 | 92 | 63 |
| 14 | 91 | 62 |

Die kristalline Verbindung weist somit eine erheblich höhere Langzeitstabilität bei Wärmeeinwirkung auf als das vorbekannte amorphe Produkt.

Beispiel 6: Trockenampullen oder Vials, enthaltend kristalline (5R,6S)-2-Aminomethyl-6-[(1R)-1-hydroxyäthyl]-2-penem-3-carbonsäure als Wirksubstanz, werden wie folgt hergestellt:

Zusammensetzung (für 1 Ampulle oder Vial):

| | |
|---|---|
| Wirksubstanz (ohne Wasser) | 0,5 g |
| Mannit | 0,05 g |

Die Wirksubstanz und das Mannit werden unter aseptischen Bedingungen abgewogen, in 10 ml-Ampullen oder 10 ml-Vials abgefüllt und die Ampullen bzw. Vials verschlossen und geprüft.

Patentansprüche (für die Vertragsstaaten BE,CH,DE,FR,GB,IT,LI,LU, NL,SE)

1. (5R,6S)-2-Aminomethyl-6-[(1R)-1-hydroxyäthyl]-2-penem-3-carbonsäure in kristalliner Form.

2. (5R,6S)-2-Aminomethyl-6-[(1R)-1-hydroxyäthyl]-2-penem-3-carbonsäure in kristalliner Form gemäss Anspruch 1, erhältlich durch Kristallisation von (5R,6S)-2-Aminomethyl-6-[(1R)-1-hydroxyäthyl]-2-penem-3-carbonsäure aus einer übersättigten Lösung in einem wasserhaltigen organischen Lösungsmittel.

3. Kristalline (5R,6S)-2-Aminomethyl-6-[(1R)-1-hydroxyäthyl]-2-penem-3-carbonsäure gemäss Anspruch 1, gekennzeichnet durch die nachfolgenden Gitterabstände (d-Werte) und relativen Linienintensitäten ihres Röntgenpulverdiagramms (Kamera nach Guinier, Strahlungsquelle: Kupfer-$K_{\alpha 1}$):

| d-Werte (Angstroem) | relative Intensität |
|---|---|
| 10.9 | sehr stark |
| 10.0 | mittel |
| 9.7 | mittel |
| 7.9 | stark |
| 7.3 | stark |
| 7.0 | stark |
| 6.7 | schwach |
| 6.3 | sehr stark |
| 5.90 | sehr schwach |
| 5.85 | sehr schwach |
| 5.60 | sehr schwach |
| 5.53 | sehr schwach |
| 5.44 | sehr schwach |
| 5.33 | sehr schwach |
| 5.01 | sehr schwach |
| 4.93 | schwach |
| 4.64 | sehr schwach |
| 4.58 | sehr schwach |
| 4.53 | stark |
| 4.42 | sehr schwach |
| 4.34 | sehr stark |

| d-Werte (Angstroem) | relative Intensität |
|---|---|
| 4.28 | sehr schwach |
| 4.23 | sehr schwach |
| 4.10 | stark |
| 4.05 | sehr schwach |
| 3.99 | mittel |
| 3.91 | schwach |
| 3.82 | mittel |
| 3.78 | mittel |
| 3.73 | stark |
| 3.65 | mittel |
| 3.59 | stark |
| 3.54 | mittel |
| 3.42 | stark |
| 3.36 | mittel |
| 3.29 | stark |
| 3.20 | mittel |
| 3.13 | mittel |
| 3.11 | stark |
| 3.03 | mittel |
| 3.00 | mittel |

4. Verfahren zur Herstellung von kristalliner (5R,6S)-2-Aminomethyl-6-[(1R)-1-hydroxyäthyl]-2-penem-3-carbonsäure, dadurch gekennzeichnet, dass man (5R,6S)-2-Aminomethyl-6-[(1R)-1-hydroxyäthyl]-2-penem-3-carbonsäure aus einer übersättigten Lösung in einem wasserhaltigen organischen Lösungsmittel zur Kristallisation bringt, das Produkt sammelt und trocknet.

5. Verfahren gemäss Anspruch 4, dadurch gekennzeichnet, dass das wasserhaltige organische Lösungsmittel eine Mischung aus Wasser und einem mit Wasser mischbaren organischen Lösungsmittel, ausgewählt aus der Gruppe niedere Alkanole mit 1 bis 4 Kohlenstoffatomen, Glykole mit 1 bis 4 Kohlenstoffatomen und deren Mono- und Di-($C_1$-$C_2$)-alkyläther, ist.

6. Verfahren gemäss Anspruch 5, dadurch gekennzeichnet, dass das wasserhaltige organische Lösungsmittel ein Mischung bestehend aus Wasser und einem der in Anspruch 5 genannten mit Wasser mischbaren organischen Lösungsmittel ist, wobei der Wassergehalt 2 bis 20 % beträgt.

7. Verfahren gemäss Anspruch 5, dadurch gekennzeichnet, dass das wasserhaltige organische Lösungsmittel eine Mischung bestehend aus Wasser und einem niederen Alkanol mit 1 bis 4 Kohlenstoffatomen ist, wobei der Wassergehalt 2 bis 20 % beträgt.

8. Verfahren gemäss Anspruch 5, dadurch gekennzeichnet, dass das wasserhaltige organische Lösungsmittel 96 %iger Aethanol ist.

9. Die nach dem Verfahren gemäss Anspruch 4 erhältliche (5R,6S)-2-Aminomethyl-6-[(1R)-1-hydroxyäthyl]-2-penem-3-carbonsäure in kristalliner Form.

10. Pharmazeutisches Präparat enthaltend (5R,6S)-2-Aminomethyl-6-[(1R)-1-hydroxyäthyl]-2-penem-3-carbonsäure in kristalliner Form.

11. Kristalline (5R,6S)-2-Aminomethyl-6-[(1R)-1-hydroxyäthyl]-2-penem-3-carbonsäure zur Anwendung in einem Verfahren zur therapeutischen Behandlung des menschlichen und tierischen Körpers.

12. Kristalline (5R,6S)-2-Aminomethyl-6-[(1R)-1-hydroxyäthyl]-2-penem-3-carbonsäure als antibiotisches Mittel.

13. Verwendung von kristalliner (5R,6S)-2-Aminomethyl-6-[(1R)-1-hydroxyäthyl]-2-penem-3-carbonsäure zur Herstellung von pharmazeutischen Präparaten.

Patentansprüche (für den Vertragsstaat AT)

1. Verfahren zur Herstellung von kristalliner (5R,6S)-2-Aminomethyl-6-[(1R)-1-hydroxyäthyl]-2-penem-3-carbonsäure, dadurch gekennzeichnet, dass man (5R,6S)-2-Aminomethyl-6-[(1R)-1-hydroxyäthyl]-2-penem-3-carbonsäure aus einer übersättigten Lösung in einem wasserhaltigen organischen Lösungsmittel zur Kristallisation bringt, das Produkt sammelt und trocknet.

2. Verfahren gemäss Anspruch 1, dadurch gekennzeichnet, dass das wasserhaltige organische Lösungsmittel eine Mischung aus Wasser und einem mit Wasser mischbaren organischen Lösungsmittel, ausgewählt aus der Gruppe niedere Alkanole mit 1 bis 4 Kohlenstoffatomen, Glykole mit 1 bis 4 Kohlenstoffatomen und deren Mono- und Di-($C_1$-$C_2$)-alkyläther, ist.

3. Verfahren gemäss Anspruch 2, dadurch gekennzeichnet, dass das wasserhaltige organische Lösungsmittel ein Mischung bestehend aus Wasser und einem der in Anspruch 2 genannten mit Wasser mischbaren organischen Lösungsmittel ist, wobei der Wassergehalt 2 bis 20 % beträgt.

4. Verfahren gemäss Anspruch 2, dadurch gekennzeichnet, dass das wasserhaltige organische Lösungsmittel eine Mischung bestehend aus Wasser und einem niederen Alkanol mit 1 bis 4 Kohlenstoffatomen ist, wobei der Wassergehalt 2 bis 20 % beträgt.

5. Verfahren gemäss Anspruch 2, dadurch gekennzeichnet, dass das wasserhaltige organische Lösungsmittel eine Mischung bestehend aus Wasser und einem niederen Alkanol mit 1 bis 4 Kohlenstoffatomen ist, wobei der Wassergehalt 2 bis 10% beträgt.

6. Verfahren gemäss Anspruch 2, dadurch gekennzeichnet, dass das organische Lösungsmittel ausgewählt wird aus der Gruppe Aethanol, n-Propanol und 2-Butanol.

7. Verfahren gemäss Anspruch 2, dadurch gekennzeichnet, dass das wasserhaltige organische Lösungsmittel 96 %iges Aethanol ist.

8. Verfahren gemäss Anspruch 1, dadurch gekennzeichnet, dass man (5R,6S)-2-Aminomethyl-6-[(1R)-1-hydroxyäthyl]-2-penem-3-carbonsäure in einem wasserhaltigen organischen Lösungsmittel bei Raumtemperatur oder bis 50°C erhöhter Temperatur löst und die Uebersättigung der Lösung durch Abkühlen der Lösung auf 0° bis 20°C bewirkt.

9. Verfahren gemäss Anspruch 1, dadurch gekennzeichnet, dass man (5R,6S)-2-Aminomethyl-6-[(1R)-1-hydroxyäthyl]-2-penem-3-carbonsäure in einem der in Anspruch 2 genannten organischen Lösungsmittel mit einem Wassergehalt von 2 bis 10 % unter Rühren digeriert.

10. Verfahren gemäss Anspruch 9, dadurch gekennzeichnet, dass man (5R,6S)-2-Aminomethyl-6-[(1R)-1-hydroxyäthyl]-2-penem-3-carbonsäure in 96 %igem Aethanol unter Rühren digeriert.

11. Verfahren gemäss Anspruch 1, dadurch gekennzeichnet, dass das Kristallisat bei normaler oder leicht erhöhter Temperatur getrocknet wird.

12. Verfahren gemäss Anspruch 1, dadurch gekennzeichnet, dass das Kristallisat unter vermindertem Druck getrocknet wird.

13. Verfahren gemäss Anspruch 1 zur Herstellung von kristalliner (5R,6S)-2-Aminomethyl-6-[(1R)-1-hydroxyäthyl]-2-penem-3-carbonsäure, gekennzeichnet durch die nachfolgenden Gitterabstände (d-Werte) und relativen Linienintensitäten ihres Röntgenpulverdiagramms (Kamera nach Guinier, Strahlungsquelle: Kupfer-$K_{\alpha 1}$):

| d-Werte (Angstroem) | relative Intensität |
|---|---|
| 10.9 | sehr stark |
| 10.0 | mittel |
| 9.7 | mittel |
| 7.9 | stark |
| 7.3 | stark |
| 7.0 | stark |
| 6.7 | schwach |
| 6.3 | sehr stark |
| 5.90 | sehr schwach |
| 5.85 | sehr schwach |
| 5.60 | sehr schwach |
| 5.53 | sehr schwach |
| 5.44 | sehr schwach |
| 5.33 | sehr schwach |
| 5.01 | sehr schwach |
| 4.93 | schwach |
| 4.64 | sehr schwach |
| 4.58 | sehr schwach |
| 4.53 | stark |
| 4.42 | sehr schwach |
| 4.34 | sehr stark |
| 4.28 | sehr schwach |
| 4.23 | sehr schwach |
| 4.10 | stark |
| 4.05 | sehr schwach |
| 3.99 | mittel |
| 3.91 | schwach |
| 3.82 | mittel |
| 3.78 | mittel |
| 3.73 | stark |
| 3.65 | mittel |
| 3.59 | stark |
| 3.54 | mittel |
| 3.42 | stark |
| 3.36 | mittel |
| 3.29 | stark |
| 3.20 | mittel |
| 3.13 | mittel |
| 3.11 | stark |
| 3.03 | mittel |
| 3.00 | mittel |

14. Verfahren zur Herstellung von pharmazeutischen Präparaten enthaltend (5R,6S)-2-Aminomethyl-6-[(1R)-1-hydroxyäthyl]-2-penem-3-carbonsäure in kristalliner Form, dadurch gekennzeichnet, dass man kristalline (5R,6S)-2-Aminomethyl-6-[(1R)-1-hydroxyäthyl]-2-penem-3-carbonsäure mit einem pharmazeutisch annehmbaren Trägerstoff mischt.

FO 7.4 UL/bg*

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0181571

Nummer der Anmeldung

EP 85 11 3841

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A- 0 013 662 (SCHERING)<br>* Seiten 4,5; Ansprüche *<br>--- | 1,10 | C 07 D 499/00<br>A 61 K 31/43//<br>C 07 D 205/08<br>C 07 F 9/65 |
| Y | TETRAHEDRON LETTERS, Band 22, Nr. 36, 1981, Seiten 3485-3488; Pergamon Press, Oxford, GB<br>V.M. GIRIJAVALLABHAN et al.: "Synthesis of optically active penems."<br>* Insgesamt *<br>--- | 1 | |
| P,Y | DE-A- 3 431 980 (CIBA-GEIGY)<br>* Ansprüche 1-23; Seiten 63,64, Beispiel 5 *<br>------- | 1,10 | |

RECHERCHIERTE SACHGEBIETE (Int. Cl.4)

C 07 D 499/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-10,12,13

Unvollständig recherchierte Patentansprüche:

Nicht recherchierte Patentansprüche: 11

Grund für die Beschränkung der Recherche:

Verfahren zur chirurgischen oder therapeutischen Behandlung des menschlichen oder tierischen Körpers (sehe Art. 52(4) des Europäisches Patentübereinkommens).

| Recherchenort | Abschlußdatum der Recherche | Prüfer |
|---|---|---|
| Den Haag | 06-02-1986 | CHOULY |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 08.82